# FASCICULE DE BREVET EUROPEEN

(11) **EP 2 991 567 B1**
(45) Date de publication et mention de la délivrance du brevet: **22.02.2017**
(21) Numéro de dépôt: 14718631.6
(22) Date de dépôt: 24.03.2014
(51) Int. Cl.: A61B 17/64, A61B 17/66

(54) **PERFECTIONNEMENT AUX FIXATEURS EXTERNES**
VERBESSERTE EXTERNE FIXATEURE
IMPROVED EXTERNAL FIXATORS

(30) Priorité: 30.04.2013 FR 1300999
(43) Date de publication de la demande: 09.03.2016
(73) Titulaire: Renard, Xavier, 91430 Vauhallan (FR)
(72) Inventeur: PELISSIER, Philippe, 33700 Merignac (FR)
(74) Mandataire: Flavenot, Bernard
(86) Numéro de dépôt international: PCT/FR2014/000061
(87) Numéro de publication internationale: WO 2014/177775

(56) Documents cités:
- FR-A1- 2 886 129
- US-A1- 2005 203 509

## Description

La présente invention concerne un perfectionnement aux fixateurs externes élastiques aptes à être montés en coopération avec deux portions d'os entre lesquelles on veut exercer soit une traction soit une distraction, qui trouvent une application particulièrement avantageuse pour traiter, par distraction, des fractures articulaires selon le principe du ligamentotaxis dans lequel la traction exercée de part et d'autre de la fracture réduit le déplacement des fragments et les maintient dans une position susceptible de favoriser le remodelage articulaire.

Le Demandeur a réalisé un fixateur externe qui fait l'objet du Brevet Européen N° 1 898 815 ci-après désigné EP-XR.

Ce fixateur externe élastique entre deux première et seconde portions d'os comporte un ressort hélicoïdal défini selon un premier axe, un premier corps, des premiers moyens pour monter en coopération le premier corps avec le ressort hélicoïdal, une première broche apte à être fixée sur la première portion d'os, la première broche étant de forme oblongue définie selon un deuxième axe, le ressort hélicoïdal étant apte à pivoter par rapport au premier corps autour du premier axe et la première broche traversant le ressort hélicoïdal, des moyens pour monter la première broche en coopération avec le premier corps de façon que le deuxième axe fasse avec le premier axe un angle non nul, un second corps comportant une embase et une partie en saillie solidaire, par une de ses extrémités, de l'embase, une des extrémités du ressort hélicoïdal étant montée en coopération autour de la partie en saillie, et des moyens pour lier le second corps avec la seconde portion d'os et ledit ressort hélicoïdal.

Le perfectionnement apporté au fixateur défini ci-dessus consiste en ce que la partie en saillie présente une section transversale croissante de façon relativement continue depuis son extrémité libre jusqu'à son extrémité solidaire de l'embase, la une des extrémités du ressort hélicoïdal ayant une section intérieure transversale supérieure à la section transversale de l'extrémité libre de la partie en saillie et inférieure à la section transversale de l'extrémité de la partie en saillie solidaire de l'embase.

D'autres caractéristiques et avantages de l'invention apparaîtront au cours de la description suivante donnée en regard des dessins annexés à titre illustratif mais nullement limitatif, dans lesquels :
La figure 1 représente une vue schématique de côté d'un mode de réalisation du fixateur externe élastique tel que défini dans le EP-XR, et
La figure 2 représente, sous forme schématique prise en combinaison avec la représentation selon la figure 1, un mode de réalisation d'une partie du fixateur externe élastique selon l'invention.

Il est tout d'abord précisé que, dans la présente description, si l'adverbe "sensiblement" est associé à un qualificatif d'un moyen donné, ce qualificatif doit être compris au sens strict ou approché.

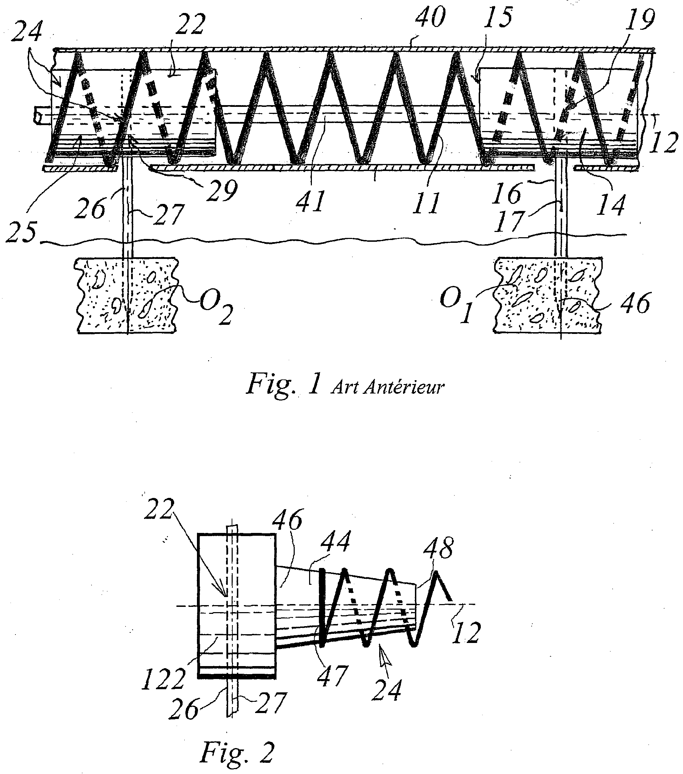

En référence à la figure 1, l'invention décrite ci-dessous est relative à un fixateur externe élastique apte à exercer une force élastique réglable en intensité entre une première et une seconde portions d'os O₁, O₂, qui trouve une application particulièrement avantageuse comme fixateur externe pour exercer une traction ou une distraction, toutes deux modulables, entre deux os ou deux fractions d'os d'un doigt de la main, par exemple entre deux phalanges.

Selon le mode de réalisation illustré sur la figure 1, le fixateur externe comporte essentiellement : un ressort hélicoïdal 11 défini selon un premier axe 12, un premier corps 14, des premiers moyens 15 pour monter en coopération ce premier corps 14 avec le ressort hélicoïdal 11 de façon que ce ressort hélicoïdal soit apte à pivoter par rapport au premier corps sensiblement autour du premier axe 12, une première broche 16 apte à être fixée sur la première portion d'os O₁, cette première broche 16 étant de forme oblongue définie selon un deuxième axe 17, et des moyens pour monter la première broche 16 en coopération avec le premier corps 14 de façon qu'elle traverse le ressort hélicoïdal 11 et que le deuxième axe 17 fasse avec le premier axe 12 un angle non nul.

Selon une réalisation avantageuse, la broche 16 est une broche osseuse bien connue en elle-même, de forme cylindrique de révolution ou analogue et dont l'extrémité 46 destinée à pénétrer dans l'os peut comporter un filetage osseux par exemple de type auto taraudant.

Quant aux moyens, 19 pour monter cette première broche 16 en coopération avec le premier corps 14, ils sont par exemple constitués d'un orifice traversant réalisé dans le corps 14 et dont la section transversale est complémentaire de celle de la broche 16, et avantageusement de moyens pour bloquer la broche dans l'orifice traversant et donc par rapport au premier corps 14, du genre vis de fixation ou analogue.

Le fixateur externe selon l'invention comporte en outre un second corps 22 et des moyens 24 pour lier ce second corps 22 avec la seconde portion d'os O₂ et le ressort hélicoïdal 11.

Dans le mode de réalisation illustré sur la figure 1, les moyens 24 pour lier le second corps 22 avec la seconde portion d'os O₂ et le ressort hélicoïdal 11 comportent des seconds moyens 25 pour monter en coopération le second corps 22 avec le ressort hélicoïdal 11 de façon que ce dernier soit apte à pivoter par rapport au second corps autour du premier axe 12, une seconde broche 26 apte à être fixée sur la seconde portion d'os O₂ et définie selon un troisième axe 27, et des moyens 29 pour monter cette seconde broche 26 en coopération avec le second corps 22 de façon qu'elle traverse le ressort hélicoïdal 11 et que le troisième axe 27 fasse avec le premier axe 12 un angle non nul.

Dans une réalisation avantageuse, le ressort hélicoïdal 11 est à spires non jointives et il est réalisé en un matériau comme de l'acier inoxydable ou analogue.

Quant au second corps 22 il comporte, en accord avec la figure 4 du EP-XR, une embase 122 et une partie en saillie 44 solidaire, par une 46 de ses extrémités, de l'embase 122, une extrémité 47 du ressort hélicoïdal 11 étant montée en coopération autour de la partie en saillie 44.

Le perfectionnement apporté au fixateur défini ci-dessus consiste en ce que la partie en saillie 44 présente une section transversale croissante de façon relativement continue depuis son extrémité libre 48 jusqu'à son extrémité 46 solidaire de l'embase 122, le ressort hélicoïdal ayant une section intérieure transversale supérieure à la section transversale de l'extrémité libre 48 de la partie en saillie et inférieure à la section transversale de l'extrémité 46 de la partie en saillie solidaire de l'embase 122.

Selon une autre caractéristique de l'invention, la partie en saillie 44 est de forme conique, de préférence de forme conique de révolution.

Le fixateur externe élastique perfectionné selon l'invention présente un avantage autre que ceux du fixateur selon le document antérieur référencé ci-dessus.

En effet, du fait notamment de la forme cylindrique de révolution de la partie en saillie 44 du fixateur de l'art antérieur, le ressort 11 pouvait, pour différentes raisons (vibrations, frottements, etc.), pivoter autour de son axe longitudinal, ayant pour conséquence une modification involontaire de l'intensité de la force appliquée entre les deux portions d'os 01, 02.

En revanche, du fait de la forme conique de la partie en saillie 44 du fixateur selon l'invention, après le réglage du ressort 11, son extrémité 47 va se coincer sur la surface conique de cette partie en saillie, ce coincement pouvant d'ailleurs être accentué par le praticien lors du réglage du fixateur en exerçant une faible rotation du ressort autour de son axe longitudinal, la dernière spire de l'extrémité 47 du ressort se dilatant alors élastiquement et augmentant son frottement sur la partie en saillie.

## Revendications

1. Fixateur externe élastique entre deux première et seconde portions d'os (O₁, O₂), comportant:
• un ressort hélicoïdal (11) défini selon un premier axe (12),
• un premier corps (14),
• des premiers moyens (15) pour monter en coopération ledit premier corps (14) avec ledit ressort hélicoïdal (11),
• une première broche (16) apte à être fixée sur ladite première portion d'os (O₁), ladite première broche (16) étant de forme oblongue définie selon un deuxième axe (17), le dit ressort hélicoïdal étant apte à pivoter par rapport audit premier corps autour du premier axe (12), et que la première broche (16) traverse ledit ressort hélicoïdal (11),
• des moyens (19) pour monter ladite première broche (16) en coopération avec ledit premier corps (14) de façon que ledit deuxième axe (17) fasse avec le premier axe (12) un angle non nul,
• un second corps (22) comportant une embase (122) et une partie en saillie (44) solidaire, par une (46) de ses extrémités, de ladite embase (122), une (47) des extrémités du ressort hélicoïdal (11) étant montée en coopération autour de ladite partie en saillie (44), et
• des moyens (24) pour lier ledit second corps (22) avec ladite seconde portion d'os (O₂) et ledit ressort hélicoïdal (11),
**caractérisé par le fait que** cette partie en saillie (44) présente une section transversale croissante de façon relativement continue depuis son extrémité libre (48) jusqu'à son extrémité (46) solidaire de ladite embase (122), la une (47) des extrémités du dit ressort hélicoïdal ayant une section intérieure transversale supérieure à la section transversale de l'extrémité libre (48) de ladite partie en saillie et inférieure à la section transversale de l'extrémité (46) de ladite partie en saillie solidaire de ladite embase (122).

2. Fixateur selon la revendication 1, **caractérisé par le fait que** la partie en saillie présentant une section transversale croissante de façon relativement continue de son extrémité libre (48) à son extrémité (46) solidaire de ladite embase (122) est de forme conique.

3. Fixateur selon la revendication 2, **caractérisé par le fait que** la partie en saillie présentant une section transversale croissante de façon relativement continue de son extrémité libre (48) à son extrémité (46) solidaire de ladite embase (122) est de forme conique de révolution.

## Patentansprüche

1. Elastischer Fixateur externe zwischen einem ersten und einem zweiten Knochenabschnitt (O₁, O₂), umfassend:
- eine Spiralfeder (11), die entlang einer ersten Achse (12) definiert ist,
- einen ersten Körper (14),
- erste Mittel (15), um den ersten Körper (14) so anzubringen, dass er mit der Spiralfeder (11) zusammenwirkt,
- einen ersten Stift (16), der geeignet ist, an dem ersten Knochenabschnitt (O₁) befestigt zu werden, wobei der erste Stift (16) eine längliche Form hat, die entlang einer zweiten Achse (17) definiert ist, wobei die Spiralfeder geeignet ist, sich im Verhältnis zu dem ersten Körper um die erste Achse (12) zu drehen und wobei der erste Stift (16) durch die Spiralfeder (11) verläuft,
- Mittel (19), um den ersten Stift (16) so anzubringen, dass er mit dem ersten Körper (14) zusammenwirkt und die zweite Achse (17) mit der ersten Achse (12) einen von null verschiedenen Winkel bildet,
- einen zweiten Körper (22), der einen Sockel (122) und einen vorstehenden Teil (44) umfasst, der an einem (46) seiner Enden mit dem Sockel (122) fest verbunden ist, wobei eines (47) der Enden der Spiralfeder (11) um den vorstehenden Teil (44) herum so angeordnet ist, dass sie zusammenwirken, und
- Mittel (24), um den zweiten Körper (22) mit dem zweiten Knochenabschnitt (O₂) und mit der Spiralfeder (11) zu verbinden,
**dadurch gekennzeichnet, dass** der vorstehende Teil (44) einen Querschnitt aufweist, der ausgehend von seinem freien Ende (48) bis zu seinem mit dem Sockel (122) fest verbundenen Ende (46) relativ kontinuierlich zunimmt, wobei eines (47) der Enden der Spiralfeder einen Innenquerschnitt besitzt, der größer als der Querschnitt des freien Endes (48) des vorstehenden Teils ist und kleiner als der Querschnitt des Endes (46) des vorstehenden Teils, das mit dem Sockel (122) fest verbunden ist, ist.

2. Fixateur nach Anspruch 1, **dadurch gekennzeichnet, dass** der vorstehende Teil, der einen Querschnitt aufweist, der von seinem freien Ende (48) zu seinem mit dem Sockel (122) fest verbundenen Ende (46) relativ kontinuierlich zunimmt, eine konische Form hat.

3. Fixateur nach Anspruch 2, **dadurch gekennzeichnet, dass** der vorstehende Teil, der einen Querschnitt aufweist, der relativ kontinuierlich von seinem freien Ende (48) zu seinem mit dem Sockel (122) fest verbundenen Ende (46) zunimmt, eine rotationssymmetrische konische Form besitzt.

## Claims

1. A resilient external fixator between first and second bone portions (O₁, O₂), comprising:
· a helical spring (11) defined along a first axis (12);
· a first body (14);
· first means (15) for mounting said first body (14) to co-operate with said helical spring (11);
· a first pin (16) suitable for being fastened to said first bone portion (O₁), said first pin (16) being oblong in shape and defined along a second axis (17), said helical spring being suitable for turning relative to the first body about the first axis (12) and the first pin (16) passes through said helical spring (11);
· means (19) for mounting said first pin (16) to co-operate with said first body (14) so that said second axis (17) forms a non-zero angle with the first axis (12);
· a second body (22) comprising a base (122) and a projecting portion (44) secured, by one (46) of its ends, to said base (122), one (47) of the ends of the helical spring (11) being mounted in co-operation around said projecting portion (44); and
· means (24) for connecting said second body (22) with said second bone portion (O₂) and said helical spring (11);
the fixator being **characterized by** the fact that said projecting portion (44) presents a cross-section that increases in relatively continuous manner from its free end (48) until it reaches its end (46) that is secured to said base (122), said one (47) of the ends of the helical spring having an inside cross-section that is greater than the cross-section of the free end (48) of said projecting portion and less than the cross-section of the end (46) of said projecting portion that is secured to said base (122).

2. A fixator according to claim 1, **characterized by** the fact that the projecting portion that presents a cross-section that increases in relatively continuous manner from its free end (48) until it reaches its end (46) that is secured to said base (122) is conical in shape.

3. A fixator according to claim 2, **characterized by** the fact that the projecting portion that presents a cross-section that increases in relatively continuous manner from its free end (48) until it reaches its end (46) that is secured to said base (122) is in the shape of a circular cone.
